# EUROPEAN PATENT SPECIFICATION

(11) **EP 2 636 373 B1**
(45) Date of publication and mention of the grant of the patent: **07.01.2015**
(21) Application number: 13157817.1
(22) Date of filing: 05.03.2013
(51) Int. Cl.: A61B 5/22, A63B 23/20

(54) **Indicator for the relaxation of the circular sphincter muscles of the anus**
Indikator für die Entspannung des Afterschließmuskels
Indicateur pour la décontraction du sphincter de l'anus

(30) Priority: 08.03.2012 IT PD20120070
(43) Date of publication of application: 11.09.2013
(73) Proprietor: A.U.S. S.R.L., 35121 Padova (IT)
(72) Inventor: Dodi, Giuseppe, 35037 Teolo PD (IT)
(74) Representative: Modiano, Micaela Nadia

(56) References cited:
- WO-A2-2012/016005
- DE-A1- 4 038 853
- FR-A1- 2 774 579
- US-A- 5 483 832

## Description

The present invention relates to an indicator of the relaxation of the circular sphincter muscles of the anus. State of the art indicators for sphincter muscles can be for example found in patent documents DE 4038853 A1 and WO 2012/016005 A2.

Constipation is generally distinguished into two forms, slow transit constipation and outlet obstruction constipation.

Slow transit constipation is a form of so-called "laziness" of the large intestine with reduction of peristalsis up to the sigmoid colon or rectum, which act as final collecting space for feces.

Outlet obstruction constipation is a disorder due to which feces, despite reaching the rectum, accumulate thereat and are expelled with difficulty, presumably caused by difficult relaxation of the pelvic muscles and in particular of the sphincter muscles.

The difficulty of rectal muscles to contract or even the total absence of contractions, caused in turn by a motor deficit of the puborectalis sling, causing a difficulty in defecation in the person affected by it, is known as puborectalis syndrome or anismus.

Dyssinergia occurs when a subject, despite pushing in order to evacuate feces, instead of releasing the sphincters and opening the anus, keeps them closed or even constricts them further, in what is known in the jargon as "paradoxal contraction" or "command inversion".

While slow transit constipation is obviated by resorting to laxatives, outlet obstruction constipation requires unpleasant local stimulation by means of suppositories, enemas, or manipulations that are either manual or with adapted instruments.

It is often necessary to combine with these unpleasant treatments a therapy for rehabilitating the pelvic muscles aimed in particular at allowing the patient to reacquire correct control of the sphincters.

So far, the execution of rehabilitation therapies is based on the use of professional instruments that have a high cost, such as for example devices for anorectal manometry and for anorectal electromyography.

An apparatus is also known for performing a test known as solid-sphere test, based on the use of a sphere to be inserted in the rectum and coupled to a dynamometer; this test allows verification of the contractility of the sphincters or their ability to relax, quantifying in grams the dynamometer values upon extraction of the sphere from the anus.

Although these systems and devices are used and appreciated due to their useful results, they have some drawbacks, such as high cost, the possibility of use only at outpatient clinics or specialized clinics and under the supervision of a trained operator, and are scarcely practical, since they require the presence of another person, in addition to the patient, for the handling of one or more of the instruments cited above.

The aim of the present invention is to provide an indicator of the relaxation of the circular sphincter muscles of the anus that is capable of obviating the cited drawbacks of the background art.

Within this aim, an object of the invention is to provide an indicator that can be used easily even by a patient on his own, at home, without the aid of a trained operator and without having to go to an outpatient clinic or to a specialized clinic.

Another object of the invention is to provide an indicator that can be used not only to evaluate a pathological state but also and above all to perform exercises for rehabilitating the correct functionality of sphincter muscles.

A further object of the invention is to provide an indicator that is cheap and can be prepared again easily for reuse.

Another object of the invention is to provide an indicator of the relaxation of the circular sphincter muscles of the anus that can be provided by means of known systems and technologies.

This aim, as well as these and other objects that will become more apparent hereinafter, are achieved by an indicator of the relaxation of the circular sphincter muscles of the anus, according to claim 1.

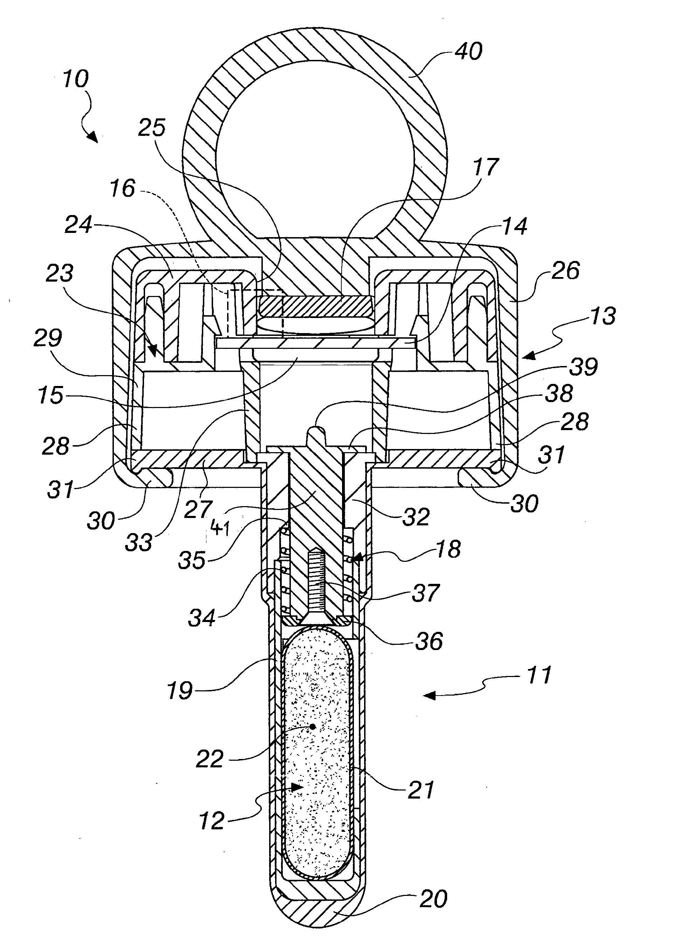

Further characteristics and advantages of the invention will become more apparent from the description of a preferred but not exclusive embodiment of the indicator of the relaxation of the circular sphincter muscles of the anus according to the invention, illustrated by way of nonlimiting example in the accompanying drawing, wherein the sole figure is a sectional side view of the indicator according to the invention.

With reference to the figure, an indicator of the relaxation of the circular sphincter muscles of the anus is generally designated by the reference numeral 10.

The indicator 10 comprises a probe shaft 11, designed to be inserted in the rectum of a user patient, said probe shaft 11 being provided with an elastically deformable body 12 that is sensitive to sphincter pressure and is described in more detail hereinafter.

The probe shaft 11 extends from a penetration-preventing wider body 13, inside which an electronic unit 14 is fitted which comprises a pressure sensor 15 that is connected to an acoustic indicator 16, and a power supply battery 17.

The pressure sensor 15 is subjected to a load, via interposed pusher means 18, by the elastically deformable body 12.

The probe shaft 11 comprises a supporting cage 19 for the elastically deformable body 12.

The cage 19 is formed for example by two symmetrical rods that are retained between an annular base and an opposite cover.

The cage 19 with the elastically deformable body 12 is covered with tight-fitting covering 20 made of rubber-like material, for example latex, or other equivalent plastic material.

The elastically deformable body 12 is constituted by a balloon 21 that is substantially cylindrical and is filled with an incompressible fluid 22, for example a gel.

The penetration-preventing wider body 13 comprises a frame 23 that supports the electronic unit 14 with pressure sensor 15 and acoustic indicator 16, and a cover 24 for protecting the electronic unit 14.

The cover has a seat 25 for the battery 17.

The frame 23 and the cover 24 are enclosed in a protective enclosure 26 made of rubber-like material, for example latex, or of equivalent plastic material.

The tight-fitting covering 20 of the probe shaft 11, in the region where the probe shaft 11 joins the penetration-preventing wider body 13, has a radially extending portion 27 which extends so as to affect the edges 28 of a perimetric wall 29 of the frame 23.

The protective enclosure 26 for the penetration-preventing wider body 13 has, at the edges 28, a folded annular portion 30 that is adapted to retain the perimetric flap 31 of the radially extended portion 27 of the tight-fitting covering 20 against the edges 28.

The pusher means 18, interposed between the elastically deformable body 12 and the pressure sensor 15, are constituted by a slider 41, which is pushed by the elastically deformable body 12, arranged so as to translate within a guiding sleeve 32, which in turn is fixed to a central collar 33 of the frame 23.

The central collar 33 opens onto the pressure sensor 15.

Elastic means for the automatic return of the slider 41 to an inactive configuration are interposed between the guiding sleeve 32 and the slider 31.

The elastic means for automatic return have a helical spring 34 arranged so as to surround the slider body.

The helical spring 34 has an end that abuts against an internal shoulder 35 of the sleeve 32 and an opposite end that abuts against the edge of a washer 36 that is screwed, by means of a screw 37, axially to the slider 41.

The slider 41 has, at the end directed toward the pressure sensor 15, a radial stroke limiting tab 38 and a central tip 39 for contact with said pressure sensor 15.

The protective enclosure 26 of the penetration-preventing wider body 13 has a grip ring 40.

The operation of the indicator according to the invention is as follows.

Once the indicator 10 has been extracted from its package or casing, the probe shaft 11 is inserted in the anus of a user.

The sphincters compress the elastically deformable body 12, which deforms and elongates, pushing the slider 31 into contact with the pressure sensor 15.

Within approximately 3 seconds after the insertion of the probe shaft 11, the indicator 10 determines a zero reference point for pressure, from which the acoustic indicator 16 is activated to emit a first sound if the pressure increases (due to contraction of the sphincters), whereas if the pressure decreases (due to relaxation of the sphincters) the acoustic indicator 16 is activated to emit a second sound that is different from the first one.

The acoustic signals emitted by the indicator 10 therefore allow the user to perceive immediately whether his attempts to control the anal muscles are successful or, on the contrary, unsuccessful.

In practice it has been found that the invention achieves the intended aim and objects.

In particular, the invention provides an indicator that can be used easily even by a patient on his own, in his own home, without the aid of a trained operator and without having to go to an outpatient clinic or to a specialized clinic.

Moreover, the invention has provided an indicator that can be used not only to evaluate a pathological condition but also and above all to perform exercises for rehabilitating the correct functionality of sphincter muscles.

Furthermore, the invention provides an indicator of the relaxation of the circular sphincter muscle of the anus that is low-cost and can be prepared again easily for reuse, since it can be easily washed and disinfected or covered with a new latex covering.

Last but not least, the invention provides an indicator of the relaxation of the circular sphincter muscles of the anus that can be provided by means of known systems and technologies.

In practice, the materials used, as long as they are compatible with the specific use, as well as the contingent shapes and dimensions, may be any according to the requirements and the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An indicator (10) of the relaxation of the circular sphincter muscles of the anus, comprising a probe shaft (11), intended to be inserted in the rectum, provided with an elastically deformable body (12) which is sensitive to sphincter pressure, said probe shaft (11) extending from a penetration-preventing wider body (13), inside which an electronic unit (14) is fitted which comprises a pressure sensor (15) connected to an acoustic indicator (16), and a power supply battery (17), said pressure sensor (15) being subjected to a load, via interposed pusher means (18), by said elastically deformable body (12), said probe shaft (11) comprising a supporting cage (19) for said elastically deformable body (12), said cage (19), together with said elastically deformable body (12), being covered with a tightly fitting covering (20) made of rubber-like material or other equivalent plastic material, said penetration-preventing wider body (13) comprising a frame (23) which supports said electronic unit (14) with pressure sensor (15) and acoustic indicator (16), and a cover (24) for protecting the electronic unit (14), said cover having a seat (25) for said battery (17), said frame (23) and said cover (24) being enclosed in a protective enclosure (26) made of rubber-like material or equivalent plastic material, said tight-fitting covering (20) of said probe shaft (11), in the region where said probe shaft (11) joins said penetration-preventing wider body (13), having a radially extended portion (27) which is extended so as to affect the edges (28) of a perimetric wall (29) of said frame (23), said protective enclosure (26) for said penetration-preventing wider body (13) having, at said edges (28), a folded annular portion (30), which is adapted to retain a perimetric flap (31) of said radially extended portion (27) of said tight-fitting covering (20) against said edges (28).

2. The indicator according to claim 1, **characterized in that** said elastically deformable body (12) is constituted by a substantially cylindrical balloon (21) which is filled with an incompressible fluid (22).

3. The indicator according to the preceding claims, **characterized in that** said pusher means (18), interposed between said elastically deformable body (12) and said pressure sensor (15), are constituted by a slider (41), which is pushed by said elastically deformable body (12), arranged so as to translate inside a guiding sleeve (32), which in turn is fixed to a central collar (33) of said frame (23), said central collar (33) opening onto said pressure sensor (15), elastic means for the automatic return of the slider (41) to an inactive configuration being interposed between said guiding sleeve (32) and said slider (41).

4. The indicator according to claim 3, **characterized in that** said elastic automatic return means have a helical spring (34) which is arranged so as to surround the slider body, said helical spring having an end which abuts against an internal shoulder (35) of said sleeve (32) and an opposite end which abuts against the edge of a washer (36) which is screwed, by means of a screw (37), axially to said slider (41).

5. The indicator according to the preceding claims 3 or 4, **characterized in that** said slider (41) has, at the end directed toward the pressure sensor (15), a radial stroke limiting tab (38) and a central tip (39) for contact with said pressure sensor (15).

6. The indicator according to the preceding claims, **characterized in that** said protective enclosure (26) of said penetration-preventing wider body (13) has a grip ring (40).

## Patentansprüche

1. Ein Indikator (10) für die Entspannung des Afterschließmuskels, der einen Sondenschaft (11) umfasst, der dazu dient, in das Rektum eingeführt zu werden, ausgestattet mit einem elastisch verformbaren Körper (12), der empfindlich gegenüber Schließmuskeldruck ist, wobei der Sondenschaft (11) sich von einem das Eindringen verhindernden breiteren Körper (13) erstreckt, in den eine Elektronikeinheit (14) montiert ist, die einen Drucksensor (15), verbunden mit einer akustischen Anzeige (16), und eine Stromversorgungsbatterie (17) umfasst, wobei der Drucksensor (15) von dem elastisch verformbaren Körper (12) über zwischengeschaltete Schubmittel (18) einer Last ausgesetzt wird, wobei der Sondenschaft (11) einen Stützkorb (19) für den elastisch verformbaren Körper (12) umfasst, wobei der Korb (19), gemeinsam mit dem elastisch verformbaren Körper (12), mit einer eng anliegenden Abdeckung (20) aus gummiartigem Material oder anderem gleichwertigem Kunststoffmaterial bedeckt ist, wobei der das Eindringen verhindernde breitere Körper (13) einen Rahmen (23) umfasst, der die Elektronikeinheit (14) mit dem Drucksensor (15) und der akustischen Anzeige (16) trägt, und eine Abdeckung (24) für den Schutz der Elektronikeinheit (14), wobei die Abdeckung einen Sitz (25) für die Batterie (17) hat, wobei der Rahmen (23) und die Abdeckung (24) in ein Schutzgehäuse (26) aus gummiartigem Material oder gleichwertigem Kunststoffmaterial eingeschlossen sind, wobei die eng anliegende Abdeckung (20) des Sondenschafts (11) in dem Bereich, in dem der Sondenschaft (11) in den das Eindringen verhindernden breiteren Körper (13) übergeht, einen radial verlängerten Abschnitt (27) hat, der so verlängert ist, dass er die Kanten (28) einer Umfangswand (29) des Rahmens (23) betrifft, wobei das Schutzgehäuse (26) für den das Eindringen verhindernden breiteren Körper (13) an den Kanten (28) einen geknickten ringförmigen Abschnitt (30) hat, der ausgebildet ist, um eine Umfangsklappe (31) des radial verlängerten Abschnitts (27) der eng anliegenden Abdeckung (20) gegen die Kanten (28) zu drücken.

2. Der Indikator gemäß Anspruch 1, **dadurch gekennzeichnet, dass** der elastisch verformbare Körper (12) aus einem im Wesentlichen zylindrischen Ballon (21) besteht, der mit einer nicht komprimierbaren Flüssigkeit (22) gefüllt ist.

3. Der Indikator gemäß den obigen Ansprüchen, **dadurch gekennzeichnet, dass** die Schubmittel (18), angeordnet zwischen dem elastisch verformbaren Körper (12) und dem Drucksensor (15), aus einem Schieber (41) bestehen, der von dem elastisch verformbaren Körper (12) geschoben wird, angeordnet, um in einer Führungshülse (32) zu translatieren, die wiederum an einer zentralen Manschette (33) des Rahmens (23) befestigt ist, wobei die zentrale Manschette (33) sich zu dem Drucksensor (15) hin öffnet, wobei elastische Mittel für die automatische Rückstellung des Schiebers (41) in eine inaktive Anordnung zwischen der Führungshülse (32) und dem Schieber (41) angeordnet sind.

4. Der Indikator gemäß Anspruch 3, **dadurch gekennzeichnet, dass** die elastischen automatischen Rückstellmittel eine Spiralfeder (34) haben, die angeordnet ist, um den Schieberkörper zu umgeben, wobei die Spiralfeder ein Ende hat, das gegen eine interne Schulter (35) der Hülse (32) anstößt, und ein gegenüberliegendes Ende, das gegen die Kante einer Unterlegscheibe (36) anstößt, die mit Hilfe einer Schraube (37) axial mit dem Schieber (41) verschraubt ist.

5. Der Indikator gemäß den obigen Ansprüchen 3 oder 4, **dadurch gekennzeichnet, dass** der Schieber (41) an dem Ende, das dem Drucksensor (15) zugewandt ist, einen radialen Hubbegrenzungsvorsprung (38) und eine zentrale Spitze (39) für den Kontakt mit dem Drucksensor (15) hat.

6. Der Indikator gemäß den obigen Ansprüchen, **dadurch gekennzeichnet, dass** das Schutzgehäuse (26) des das Eindringen verhindernden breiteren Körpers (13) einen Greifring (40) hat.

## Revendications

1. Indicateur (10) du relâchement des muscles circulaires du sphincter de l'anus, comprenant une tige de sonde (11), destinée à être insérée dans le rectum, dotée d'un corps déformable de manière élastique (12) qui est sensible à la pression du sphincter, ladite tige de sonde (11) s'étendant à partir d'un corps plus large qui empêche sa pénétration (13), à l'intérieur duquel est placée une unité électronique (14) qui comprend un capteur de pression (15) relié à un indicateur acoustique (16), et une batterie d'alimentation électrique (17), ledit capteur de pression (15) étant soumis à une charge, par l'intermédiaire de moyens formant poussoir interposés (18), par ledit corps déformable de manière élastique (12), ladite tige de sonde (11) comprenant une cage de support (19) dudit corps déformable de manière élastique (12), ladite cage (19), ainsi que ledit corps déformable de manière élastique (12), étant recouverts par un recouvrement ajusté au plus près (20) réalisé dans un matériau similaire à du caoutchouc ou dans tout autre matériau de matière plastique similaire, ledit corps plus large qui empêche sa pénétration (13) comprenant un bâti (23) qui supporte ladite unité électronique (14) dotée du capteur de pression (15) et de l'indicateur acoustique (16), et un couvercle (24) destiné à protéger l'unité électronique (14), ledit couvercle présentant un siège (25) destiné à recevoir ladite batterie (17), ledit bâti (23) et ledit couvercle (24) étant inclus dans une enceinte de protection (26) réalisée dans un matériau similaire à du caoutchouc ou dans tout autre matériau de matière plastique similaire, ledit recouvrement ajusté au plus près (20) de ladite tige de sonde (11), dans la région où ladite tige de sonde (11) rejoint ledit corps plus large qui empêche sa pénétration (13), présentant une partie étendue de manière radiale (27) qui est étendue de façon à affecter les bords (28) d'une paroi périmétrique (29) dudit bâti (23), ladite enceinte de protection (26) dudit corps plus large qui empêche sa pénétration (13) présentant, au niveau desdits bords (28), une partie annulaire pliée (30), qui est adaptée de façon à retenir un volet périmétrique (31) de ladite partie étendue de manière radiale (27) dudit recouvrement ajusté au plus près (20) contre lesdits bords (28).

2. Indicateur selon la revendication 1, **caractérisé en ce que** ledit corps déformable de manière élastique (12) est constitué par un ballonnet sensiblement cylindrique (21) qui est rempli avec un fluide incompressible (22).

3. Indicateur selon les revendications précédentes, **caractérisé en ce que** lesdits moyens formant poussoir (18), interposés entre ledit corps déformable de manière élastique (12) et ledit capteur de pression (15), sont constitués par un coulisseau (41), qui est poussé par ledit corps déformable de manière élastique (12), agencé de façon à se déplacer à l'intérieur d'un manchon de guidage (32), qui est fixé à son tour sur un collier central (33) dudit bâti (23), ledit collier central (33) s'ouvrant sur ledit capteur de pression (15), des moyens élastiques destinés à permettre le retour automatique du coulisseau (41) jusqu'à une configuration inactive, étant interposés entre ledit manchon de guidage (32) et ledit coulisseau (41).

4. Indicateur selon la revendication 3, **caractérisé en ce que** lesdits moyens élastiques de retour automatique présentent un ressort hélicoïdal (34) qui est agencé de façon à entourer le corps du coulisseau, ledit ressort hélicoïdal présentant une extrémité qui vient en butée contre un épaulement intérieur (35) dudit manchon (32), et une extrémité opposée qui vient en butée contre le bord d'une rondelle (36) qui est vissée, à l'aide d'une vis (37), de manière axiale sur ledit coulisseau (41).

5. Indicateur selon les revendications précédentes 3 ou 4, **caractérisé en ce que** ledit coulisseau (41) présente, au niveau de l'extrémité dirigée vers le capteur de pression (15), une patte de limitation de la course radiale (38) et un bout central (39) destiné à entrer en contact avec ledit capteur de pression (15).

6. Indicateur selon les revendications précédentes, **caractérisé en ce que** ladite enceinte de protection (26) dudit corps plus large qui empêche sa pénétration (13) présente un anneau de préhension (40).
